# EUROPÄISCHE PATENTSCHRIFT

(11) **EP 1 270 039 B1**
(45) Veröffentlichungstag und Bekanntmachung des Hinweises auf die Patenterteilung: **08.11.2006**
(21) Anmeldenummer: 02011910.3
(22) Anmeldetag: 29.05.2002
(51) Int. Cl.: A61M 25/00

(54) **Katheter**
Catheter
Cathéter

(30) Priorität: 19.06.2001 DE 20110121 U
(43) Veröffentlichungstag der Anmeldung: 02.01.2003
(73) Patentinhaber: B. BRAUN MELSUNGEN AG, 34212 Melsungen (DE)
(72) Erfinder: Goebel, Udo, 34212 Melsungen (DE); Sippel, Martin, 34212 Melsungen (DE)
(74) Vertreter: Selting, Günther

(56) Entgegenhaltungen:
- EP-A- 0 064 212
- EP-A- 0 452 123
- WO-A-99/42156
- US-A- 4 639 252
- US-A- 5 234 406
- US-A- 5 830 196
- US-B1- 6 221 059

## Beschreibung

Die Erfindung betrifft einen Katheterset für die Regionalanästhesie, bestehend aus einem langgestreckten Schlauch aus in Längsrichtung über die Schlauchlänge durchgehend gleichem Material, mit einem proximalen Schlauchabschnitt und einem distalen Schlauchabschnitt, wobei der distale Schlauchabschnitt relativ zu dem proximalen Schlauchabschnitt einen verringerten Außendurchmesser aufweist, und einer Stahlkanüle.

Ein Katheter ist ein Schlauch, der mit einem Teil seiner Länge in den Körper eingeführt wird, um eine Flüssigkeits- oder Gasverbindung herzustellen und beispielsweise Medikamente dem Körper zuzufügen oder Flüssigkeit aus dem Körper zu entfernen.

Hierzu muss der Schlauch spezielle Anforderungen erfüllen. Er muss beispielsweise eine hinreichende Steifigkeit haben, um in den Körper hinein vorgeschoben zu werden. Andererseits muss die Katheterspitze eine gewisse Weichheit haben, um Blutgefäße oder andere Körperteile nicht zu verletzen.

Ein Katheterset nach dem Oberbegriff des Patentanspruchs 1 ist beschrieben in U.S.-A. 5,234,406. Dieser Katheterset besteht aus einem zweiteiligen Katheter, der einen proximalen Abschnitt großen Durchmessers und einen distalen Abschnitt kleinen Durchmessers aufweist. Der distale Abschnitt wird durch eine Kanüle hindurch in den Epiduralraum hinein vorgeschoben. Anschließend wird die Kanüle über den verlegten Katheterabschnitt nach hinten abgezogen. Die Weite der Kanüle ist nur so groß, dass der distale Schlauchabschnitt hindurchpasst. Daher kann der distale Schlauchabschnitt von dem dickeren proximalen Schlauchabschnitt gelöst werden, um die Kanüle entfernen zu können. Der distale Abschnitt weist eine konisch zulaufende distale Spitze auf, welche aus dem Ende der kanüle herausragen kann.

In EP 0 437 291 B1 ist ein Verfahren zur Herstellung einer weichen Katheterspitze bekannt, bei dem im Katheterschlauch eine Spitze aus einem Material angespritzt wird, welches weicher ist als das Kathetermaterial. Die Katheterspitze verjüngt sich zum distalen Ende hin konisch. Dadurch erhält man einen Katheter, dessen Katheterspitze weich ist, so dass das Risiko innerer Verletzungen vermieden wird.

Aus EP 0 452 123 B1 ist ein Katheter bekannt, der aus einem radiopaken halogenierten Polyurethan besteht. Dieser Katheter kann mehrere Schichten aus unterschiedlichen Materialien aufweisen.

Ein Venenverweilkatheter aus Polyurethan mit konischer Spitze ist bekannt aus EP 0 523 928 A2. Der Venenverweilkatheter, der mit einer eingeschobenen Stahlkanüle in den Patientenkörper eingebracht wird, besteht aus Polyurethan. Dieses Material ist biokompatibel und ist als optisch transparentes radiopakes Material verfügbar. Es bietet eine glatte Oberfläche und eine hohe Knickfestigkeit.

Katheter mit einer weichen Spitze werden auch im Bereich der Zentralen Venenpunktion eingesetzt. Die Weichspitze soll Venenperforationen und Verletzungen der Intima reduzieren. Ferner werden Katheter mit weicher Spitze in der Epiduralanästhesie benutzt, um das Auftreten von Gefäß- oder Duraverletzungen sowie Parästhesien zu verringern. In der Kardiologie werden Katheter mit unterschiedlich gestalteten Spitzen eingesetzt, um in die verschieden geformten Venen und Arterien zu gelangen.

Der Erfindung liegt die Aufgabe zugrunde, ein Katheterset für die Regionalanästhesie zu schaffen, der das Einführen des Katheters in den Patientenkörper erleichtert und der außerdem mit geringem Aufwand hergestellt werden kann.

Der erfindungsgemäße Katheterset weist die Merkmale des Patentanspruchs 1 auf. Hiernach ist der dickere proximale Schlauchabschnitt des Katheters in die Stahlkanüle einschiebbar, derart, dass der dünnere distale Schlauchabschnitt mit seiner Länge aus dem Ende der Stahlkanüle herausragen kann.

Der verringerte Durchmesser des distalen Schlauchabschnitts kann durch Strecken oder Ausziehen des Kathetermaterials hervorgerufen werden, wobei der Katheter gereckt und dadurch plastisch verformt wird. Hierbei wird die Länge des distalen Schlauchabschnitts vergrößert und durch Querkontraktion wird der Außendurchmesser verringert. Der Schlauch besteht durchgehend aus gleichem Schlauchmaterial und er wird lediglich durch Recken verformt. Daher ist es nicht erforderlich zwei unterschiedliche Materialen miteinander zu verbinden.

Durch das Ausziehen des distalen Schlauchabschnitts erhält dieser Schlauchabschnitt eine größere Weichheit und Verformbarkeit. Sowohl das Vorschieben über einen Führungsdraht als auch das Hindurchschieben durch eine Kanüle werden erleichtert.

Die Erfindung schafft einen Katheter mit einem hochflexiblen weichen distalen Schlauchabschnitt und einem stabilen proximalen Schlauchabschnitt mit guter Verschiebbarkeit (Pushability) unter Beibehaltung des Grundmaterials des Katheters.

Nach dem Recken kann eine Nachbearbeitung des distalen Schlauchabschnitts erfolgen, der auf die erforderliche Länge geschnitten und/ oder mit Verrundungen versehen wird. Auch können Löcher an dem distalen Schlauchabschnitt angebracht werden.

Das Prinzip der vorliegenden Erfindung wird bevorzugt angewandt für Regionalanästhesiekatheter, insbesondere für Spinalanästhesiekatheter, die in das Rückenmark eingeführt werden, Epiduralanästhesiekatheter, die in den neben dem Rückenmark liegenden Epiduralraum verlegt werden, und Plexuskathetern. Diese Katheter werden in Verbindung mit einer zugehörigen Regionalanästhesiekanüle benutzt, insbesondere einer Spinalanästhesiekanüle, einer Epiduralkanüle oder einer Plexuskanüle.

Der verjüngte distale Schlauchabschnitt ist vorzugsweise im Wesentlichen zylindrisch und er hat eine Länge, die mindestens gleich dem Fünffachen, vorzugsweise mindestens gleich dem Zehnfachen des Außendurchmessers des proximalen Schlauchabschnitts ist. Dies bedeutet, dass nicht lediglich die Katheterspitze im Durchmesser verringert ist sondern der gesamte distale Schlauchabschnitt.

Im Folgenden werden unter Bezugnahme auf die Zeichnungen Ausführungsbeispiele der Erfindung näher erläutert.

Es zeigen:
- Fig. 1: einen Längsschnitt durch einen Epiduralkatheter nach der vorliegenden Erfindung mit geschlossener Katheterspitze,
- Fig. 2: eine Verwendung des Katheters nach Fig. 1 in Verbindung mit einer Tuohy-Kanüle,
- Fig. 3: als Beispiel, welches nicht unter den Schutzbereich der Ansprüche fällt einen Längsschnitt durch einen zentralvenösen Katheter, und

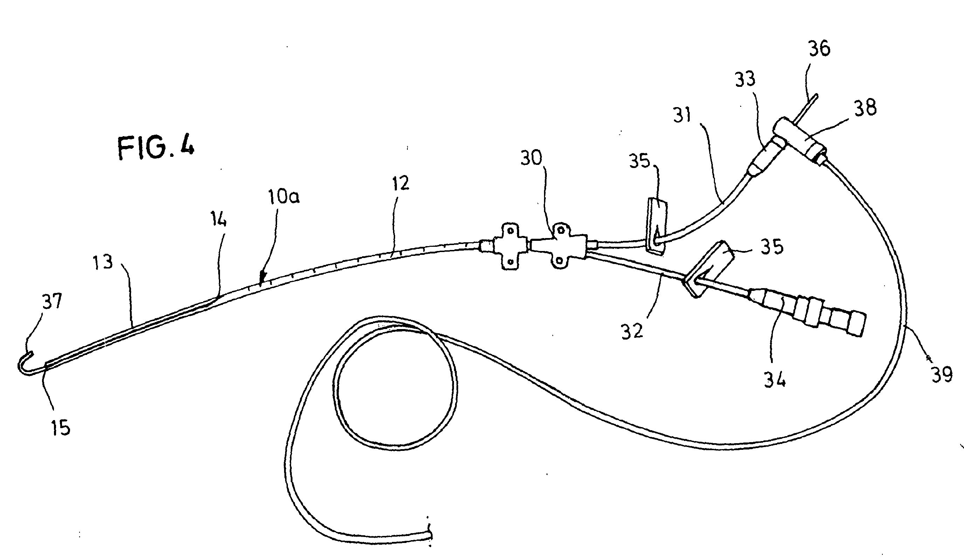

- Fig. 4: eine Gesamtdarstellung des zentralvenösen Katheters, der nicht unter den Schutzbereich der Ansprüche fällt.

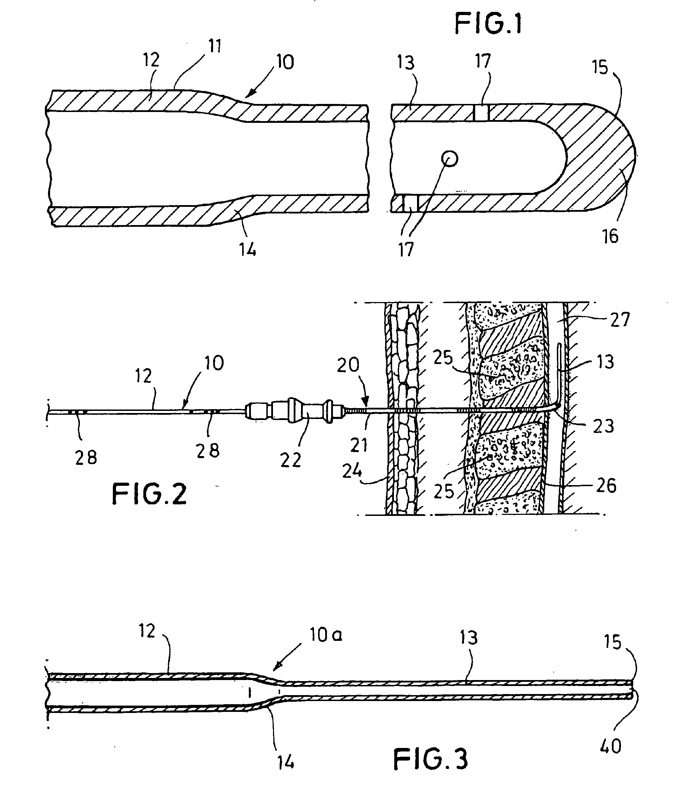

Der in Fig. 1 dargestellte Katheter 10 ist ein Epiduralkatheter, der in der Regionalanästhesie benutzt wird, um ein Lokalanästhetikum z.B. in den Epiduralraum zu injizieren. Der Katheter 10 besteht aus einem langgestreckten Schlauch 11 aus über die Länge durchgehend gleichem Material. Der Schlauch 11 weist einen proximalen Schlauchabschnitt 12 und einen distalen Schlauchabschnitt 13 auf. Der Katheter hat eine Länge von insgesamt 1000 mm. Der Außendurchmesser des proximalen Schlauchabschnitts 12 beträgt 0,85 mm und der Innendurchmesser 0,45 mm. Diese Angaben sind jedoch nur als Beispiel zu verstehen. Selbstverständlich sind auch andere Abmessungen möglich.

Der distale Schlauchabschnitt 13 hat eine Länge von mindestens 10 mm. Der Außendurchmesser ist im distalen Schlauchabschnitt 13 auf etwa 80 % des Außendurchmessers des proximalen Schlauchabschnitts reduziert. Diese Reduktion erfolgt durch Reckung, wobei ein Übergangsbereich 14 entsteht. Das Recken erfolgt durch Langziehen des Schlauchs, dessen proximaler Schlauchabschnitt 12 durch eine Klemmvorrichtung festgehalten wird, während der distale Schlauchabschnitt 13 in die Länge gezogen wird. Beim Recken kann erforderlichenfalls ein entsprechender Dorn benutzt werden. Beim Langziehen des distalen Schlauchabschnitts 13 verringert sich dessen Wandstärke durch Querkontraktion. Am distalen Ende des distalen Schlauchabschnitts 13 befindet sich die Katheterspitze 15, die bei dem vorliegenden Ausführungsbeispiel durch eine aus dem Kathetermaterial bestehende runde Abschlusskappe 16 gebildet wird. Alternativ hierzu ist auch eine zentrale Öffnung an der Katheterspitze möglich. Bei dem Katheter 10 nach Fig. 1 sind in der Nähe der Katheterspitze an den distalen Schlauchabschnitten seitliche Öffnungen 17 angebracht, durch die Flüssigkeit aus dem Katheter austreten kann.

In Fig. 2 ist der Gebrauch des Katheters 10 als Epiduralkatheter dargestellt. Der Katheter 10 wird in Verbindung mit einer Tuohy-Kanüle 20 benutzt. Diese weist eine Stahlkanüle 21 auf, die an ihrem proximalen Ende mit einem Kanülenansatz 22 versehen ist. Das distale Kanülenende ist mit einer Biegung 23 versehen. Die Kanüle 20 wird in der in Fig. 2 dargestellten Weise durch die Haut 24 hindurch, zwischen den Wirbeln 25 der Wirbelsäule hindurch, durch das Ligamentum flavum 26 in den Epiduralraum 27 vorgeschoben. Dann wird durch die Kanüle 20 hindurch der Katheter 10 geschoben, der durch das gebogene Kanülenende 23 abgewinkelt wird und im Epiduralraum 27 vorgeschoben wird. Danach wird die Kanüle 20 von dem Katheter 10 abgezogen und durch den Katheter kann ein Lokalanästhetikum in den Epiduralraum injiziert werden. Der verjüngte distale Katheterabschnitt 13 hat bei diesem Ausführungsbeispiel eine Länge von etwa 5 cm. Diese Länge entspricht derjenigen Länge, die über das Kanülenende 23 hinaus in den Epiduralraum hineinragt. An dem proximalen Schlauchabschnitt 12 sind Markierungen 28 angebracht, mit denen die Einschubtiefe des Katheters bestimmt werden kann.

Fig. 3 zeigt als Beispiel einen Katheter 10a, der als zentralvenöser Katheter ausgebildet ist und nicht unter den Schutzbereich der Ansprüche fällt. Dieser Katheter wird durch eine Zentralvene hindurch bis in die unmittelbare Nähe des Herzens verlegt, wobei die Katheterspitze 15 zunächst unter EKG-Lagekontrolle bis in den rechten Vorhof vorgeschoben und anschließend in die Hohlvene hinein zurückgezogen wird. In die Hohlvene wird dann ein Medikament durch den Katheter injiziert. Der Katheter enthält einen Führungsdraht, dessen Spitze an der Katheterspitze 15 austritt. Die Ableitung der EKG-Signale erfolgt über den Führungsdraht.

Die Gesamtlänge des Katheters 10a beträgt bei einem zentralvenösen Katheter etwa 15 cm.

Der Katheter 10a weist einen proximalen Schlauchabschnitt 12 und einen dem gegenüber verjüngten distalen Schlauchabschnitt 13 auf, die beide durchgehend aus demselben Material bestehen. Der distale Schlauchabschnitt 13 hat eine Länge von mindestens 10 cm.

Der Katheter 10a ist mit einem Y-Stück 30 verbunden, von dem zwei Abzweigungen 31 und 32 abgehen. Die Abzweigung 31 ist mit einem Katheteransatz 33 verbunden und die Abzweigung 32 ist mit einem Katheteransatz 34 verbunden. Jede der Abzweigungen 31 und 32 weist eine Schlauchklemme 35 auf. Durch die Abzweigung 31 und den Katheter 10a führt ein Führungsdraht 36 hindurch, der am distalen Ende eine leicht verformbare gebogene Spitze 37 aufweist. Die Spitze 37 ragt aus der Katheterspitze 15 heraus. An das aus dem Katheteransatz 33 herausragende distale Ende des Führungsdrahts 36 wird mit einer Kontaktklemme 38 ein Kabel 39 angeschlossen, welches mit einem EKG-Anzeigegerät verbunden werden kann. Auf diese Weise kann durch EKG-Kontrolle festgestellt werden, ob die Katheterspitze sich im rechten Vorhof oder in der Hohlvene befindet.

Der Führungsdraht 36 hat einen solchen Durchmesser, dass er ohne wesentliche Reibung durch den distalen Schlauchabschnitt 13 geschoben werden kann. Er tritt an der zentralen Öffnung an der Katheterspitze 15 aus. Die Öffnungskanten sind gerundet.

Der Katheter 10a, der bei dem Beispiel ein einziges Lumen hat, kann auch mehrlumig ausgeführt sein. Durch das Recken des distalen Schlauchabschnitts werden sämtliche Lumen im Durchmesser verkleinert.

Der Katheter besteht durchgehend über seine gesamte Länge aus demselben Material. Er kann allerdings auch zwei- oder mehrschichtig ausgebildet sein und ein Innenrohr und ein Außenrohr aufweisen, wobei der Katheter, insbesondere durch Koextrusion beider Rohre hergestellt werden kann.

Für einen Epiduralkatheter eignet sich besonders die Materialkombination PA-PU, wobei das Innenrohr aus Polyamid und das Außenrohr aus Polyurethan besteht. Für einen zentralvenösen Katheter eignet sich besonders eine Materialkombination PU-PU aus unterschiedlichem Polyurethanen und für eine Venenverweilkanüle eine Materialkombination PA-PA aus unterschiedlichen Polyamiden.

## Patentansprüche

1. Katheterset für die Regionalanästhesie bestehend aus einem langgestreckten Schlauch (11) aus in Längsrichtung über die Schlauchlänge durchgehend gleichem Material, mit einem proximalen Schlauchabschnitt (12) und einem distalen Schlauchabschnitt (13), wobei der distale Schlauchabschnitt (13) relativ zu dem proximalen Schlauchabschnitt (12) einen verringerten Außendurchmesser aufweist, und einer Stahlkanüle (21), wobei
der dickere proximale Schlauchabschnitt (12) in die Stahlkanüle (21) einschiebbar ist, derart, dass der dünnere distale Schlauchabschnitt (13) mit seiner Länge aus dem Ende der Stahlkanüle (21) hinausragen kann,
**dadurch gekennzeichnet, dass** der distale Schlauchabschnitt (13) zylindrisch ist.

2. Katheterset nach Anspruch 1 **dadurch gekennzeichnet, dass** der distale Schlauchabschnitt (13) eine Länge hat, die mindestens gleich dem Fünffachen, vorzugsweise mindestens gleich dem Zehnfachen, des Außendurchmessers des proximalen Schlauchabschnitts (12) ist.

3. Katheterset nach einem der Ansprüche 1 - 2, **dadurch gekennzeichnet, dass** die Katheterspitze (15) eine zentrale Öffnung (40) mit verrundeter Kante aufweist.

4. Katheterset nach einem der Ansprüche 1 - 2, **dadurch gekennzeichnet, dass** die Katheterspitze (15) eine aus dem Kathetermaterial bestehende runde Abschlusskappe (16) aufweist.

5. Katheterset nach einem der Ansprüche 1 - 4, **dadurch gekennzeichnet, dass** der Außendurchmesser des distalen Schlauchabschnitts (13) 50 - 90 % des Außendurchmessers des proximalen Schlauchabschnitts (12) beträgt.

6. Katheterset nach einem der Ansprüche 1 - 5, **dadurch gekennzeichnet, dass** der Schlauch (11) aus einem Verbundmaterial mit einem Außenrohr aus PU und einem Innenrohr aus PU oder PA besteht.

## Claims

1. A catheter set for regional anesthesia, comprising an elongate tube (11) made from a material identical throughout the length of the tube (11) in longitudinal direction and having a proximal tube portion (12) and a distal tube portion 13), the distal tube portion (13) having a reduced outer diameter relative to the proximal tube portion (12), and comprising a steel cannula (21), the thicker proximal tube portion (12) being adapted to be inserted into the steel cannula (21) in such a manner that the thinner distal tube portion (13) with its length can project from the end of the steel cannula (21),
**characterized in**
**that** the distal tube portion (13) is cylindrical.

2. The catheter set according to claim 1, **characterized in that** the distal tube portion (13) has a length at least five times and preferably at least ten times as large as the outer diameter of the proximal tube portion (12).

3. The catheter set according to any one of claims 1-2, **characterized in that** the catheter tip (15) has a central opening (40) with a rounded edge.

4. The catheter set according to any one of claims 1-2, **characterized in that** the catheter tip (15) comprises a round end cap (16) made from the catheter material.

5. The catheter set according to any one of claims 1-4, **characterized in that** the outer diameter of the distal tube portion (13) is 50% - 90% of the outer diameter of the proximal tube portion (12).

6. The catheter set according to any one of claims 1-5, **characterized in that** the tube (11) is made of a composite material with an outer tube made of PU and an inner tube made of PU or PA.

## Revendications

1. Ensemble cathéter pour l'anesthésie locale, constitué d'un tube long (11) fabriqué dans un même matériau sur toute la longueur du tube dans le sens longitudinal, comportant un tronçon de tube proximal (12) et un tronçon de tube distal (13), le tronçon de tube distal (13) présentant, par rapport au tronçon de tube proximal (12), un diamètre extérieur réduit, et une canule en acier (21), dans lequel le tronçon de tube proximal (12) plus épais peut être introduit dans la canule en acier (21) de manière à ce que le tronçon de tube distal (13) plus mince puisse dépasser avec sa longueur de l'extrémité de la canule en acier (21), **caractérisé en ce que** le tronçon de tube distal (13) est cylindrique.

2. Ensemble cathéter selon la revendication 1, **caractérisé en ce que** le tronçon de tube distal (13) est au moins cinq fois plus long, de préférence au moins dix fois plus long que le diamètre extérieur du tronçon de tube proximal (12).

3. Ensemble cathéter selon l'une quelconque des revendications 1-2, **caractérisé en ce que** la pointe (15) du cathéter présente une ouverture centrale (40) ayant une arête arrondie.

4. Ensemble cathéter selon l'une quelconque des revendications 1-2, **caractérisé en ce que** la pointe (15) du cathéter présente un capuchon (16) rond fabriqué dans le même matériau que le cathéter.

5. Ensemble cathéter selon l'une quelconque des revendications 1-4, **caractérisé en ce que** le diamètre extérieur du tronçon de tube distal (13) représente 50 à 90 % du diamètre extérieur du tronçon de tube proximal (12).

6. Ensemble cathéter selon l'une quelconque des revendications 1-5, **caractérisé en ce que** le tube (11) est constitué d'un matériau composite comportant un tube extérieur en PU et un tube intérieur en PU.
